## Europäisches Patentamt
## European Patent Office
## Office européen des brevets

(19)

(11) Numéro de publication: **0 403 384**
**A2**

(12) **DEMANDE DE BREVET EUROPEEN**

(21) Numéro de dépôt: **90401650.8**

(22) Date de dépôt: **14.06.90**

(51) Int. Cl.⁵: **C12Q 1/70, C12Q 1/68, C07H 21/04**

(30) Priorité: **15.06.89 FR 8907970**

(43) Date de publication de la demande:
**19.12.90 Bulletin 90/51**

(84) Etats contractants désignés:
**BE CH DE DK ES FR GB IT LI NL**

(71) Demandeur: **SOCIETE EUROPEENNE DE BIOTECHNOLOGIE (EN ABREGE EUROGENTEC) SOCIETE ANONYME DE DROIT BELGE**
**13 rue Vignoble**
**Esneux(BE)**

Demandeur: **RHôNE MERIEUX**
**17, rue Bourgelat**
**F-69002 Lyon(FR)**

(72) Inventeur: **Vaira, Dolores**
**Cité des Mineurs 7**
**B-4601 Chaudfontaine(BE)**
Inventeur: **Renard, André, Jean, Joseph**
**Av. des Cerfs, 37**
**B-4000 Liège(BE)**
Inventeur: **Allaer, Didier, Georges, Jean-Marie**
**rue des Sabotiers 1**
**B-4140 Jehay(BE)**
Inventeur: **Rossius, Michel, Thierry, Jean-François**
**rue Alleur 100**
**B-4000 Liège(BE)**

(74) Mandataire: **Bernasconi, Jean et al**
**CABINET LEMOINE ET BERNASCONI 13,**
**Boulevard des Batignolles**
**F-75008 Paris(FR)**

(54) **Procédé de recherche et de préparation de sondes chez les pestivirus, oligonucléotides et sondes obtenues et procédé de détection des pestivirus.**

(57) Dans le procédé de recherche et de préparation de sondes communes et/ou spécifiques des variétés de Pestivirus, on amplifie une zone d'environ 110 à 115 bases située vers l'extrémité 5' sur le génome d'un Pestivirus dans la région non codante correspondant à la région située entre les bases 200 et 310 du génome du virus BVD Osloss à l'aide d'amorces convergentes s hybridant sensiblement au niveau des extrémités de cette zone non codante, on séquence la zone amplifiée et, par comparaison aux zones similaires déjà séquencées d'autres variétés de Pestivirus, on recherche les séquences oligonucléotidiques homologues et/ou spécifiques des sondes.

## Procédé de recherche et de préparation de sondes chez les Pestivirus, oligonucléotides et sondes obtenus et procédé de détection des Pestivirus.

L'invention a trait à un procédé de recherche et de préparation de sondes communes et/ou spécifiques des variétés de Pestivirus, à de nouveaux oligonucléotides destinés notamment à entrer dans la composition de sondes pour la détection de virus du genre Pestivirus par hybridation moléculaire ADN-ARN ou ADN-ADN in vitro, aux sondes obtenues ainsi qu'à des procédés de détection mettant en oeuvre lesdites sondes selon l'invention.

Dans la classification actuelle, le genre Pestivirus comprend essentiellement trois espèces, à savoir le virus de la diarrhée bovine (BVD), le virus de la peste porcine et le virus de la maladie de Border. Ces différentes espèces se divisent elles-mêmes en de nombreuses variétés ou sous-espèces, dont bien peu sont connues de manière précise.

Les affections virales causées par ces différents virus présentent des symptômes qu'il est souvent difficile de distinguer de ceux accompagnant d'autres maladies à virus des animaux, ce qui en rend difficile le diagnostic.

Par ailleurs, les risques de contamination du sérum rendent indispensable la détection des Pestivirus dans le sérum utilisé dans la préparation de produits pour animaux, de vaccins, etc.

Cependant, les techniques de diagnostic et les techniques de détection traditionnelles, par exemple ELISA, se révèlent parfois insuffisantes.

On connaît depuis longtemps la technique de l'hybridation moléculaire dans laquelle on utilise une petite séquence d'ADN ou d'ARN marquée (sonde) pour détecter des séquences homologues ou complémentaires.

Dans le cas présent, la difficulté provient notamment de la grande diversité des variétés ou sous-espèces alors qu'il est indispensable d'aboutir à un moyen de détection et éventuellement d'identification de la plupart, et si possible, de tous les Pestivirus.

L'invention a donc pour objectif de fournir un procédé de recherche et de préparation de sondes communes et/ou spécifiques des variétés de Pestivirus.

Un autre objectif de l'invention est de fournir des oligonucléotides de séquence complémentaire de séquences communes à tous les Pestivirus et de séquences spécifiques de chaque espèce et de chaque sous-espèce, susceptibles d'être utilisés comme ou incorporés dans des sondes d'hybridation moléculaire, ou des mélanges de telles sondes, destinés à la détection et à l'idenfication des virus du genre Pestivirus.

Un autre objectif encore de l'invention est de fournir un procédé de détection et/ou d'identification des Pestivirus dans un échantillon, qui soit simple, facile à mettre en oeuvre, rapide et fiable et qui soit susceptible d'être, au moins en partie, automatisé.

La déposante a découvert qu'il était particulièrement intéressant de séquencer une partie du génome des variétés de Pestivirus située dans la même zone sur les génomes, à savoir sur l'extrémité 5', dans la région non codante et, plus précisément une partie d'environ 110 bases correspondant à la partie située entre la base 200 et la base 310 du génome de BVD Osloss.

La déposante a notamment séquencé cette partie (110 bases) dans le génome de 19 variétés de Pestivirus (voir leur origine et leur biotype en annexe) :
- 12 variétés de virus BVD,
- 3 variétés de virus de la maladie de Border, et
- 4 variétés de virus de la peste porcine,
étant entendu que les séquences complètes de 2 variétés de virus BVD sont déjà connues (BVD variété Osloss C : EP-A-0.208.672 et BVD Nadl, Collet et al., Virology, 165, 1988, P. 191-199).

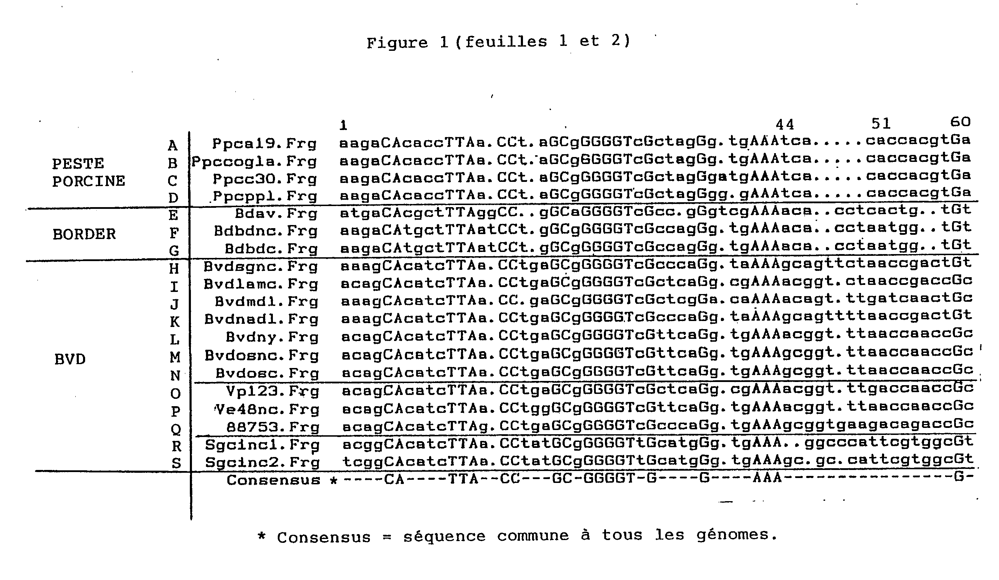

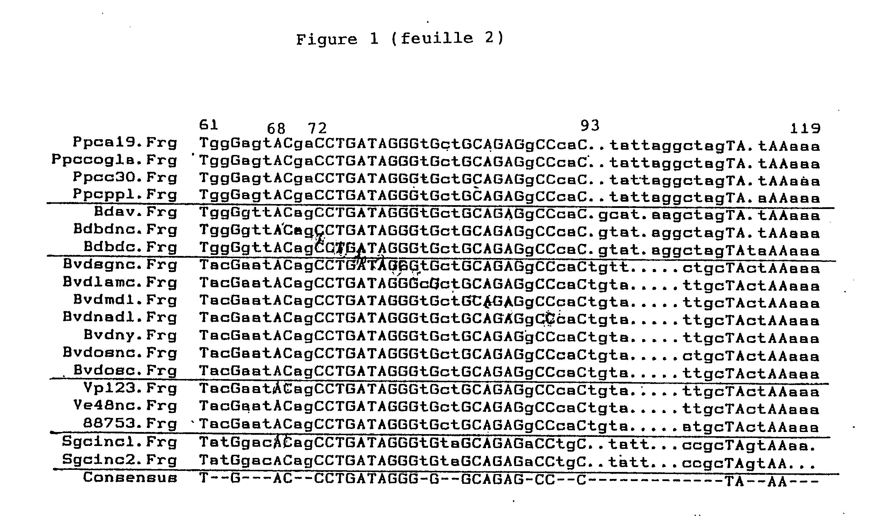

Une étude comparative de ces séquences à permis de déterminer une séquence de 20 bases (bases 93 à 72 à la figure 1) sensiblement commune à tous les Pestivirus et, également, des séquences spécifiques de chaque espèce ainsi que des séquences spécifiques de sous-espèces.

L'invention a pour objet un procédé de recherche et de préparation de sondes communes et/ou spécifiques des variétés de Pestivirus, caractérisé en ce que l'on amplifie une zone d'environ 110 à 115 bases située vers l'extrémité 5' sur le génome d'un Pestivirus dans la région non codante correspondant à la région située entre les bases 200 et 310 du génome du virus BVD Osloss à l'aide d'amorces convergentes s'hybridant sensiblement au niveau des extrémités de cette zone non codante, que l'on séquence la zone amplifiée et que, par comparaison aux zones similaires déjà séquencées d'autres variétés de Pestivirus, on recherche les séquences oligonucléotidiques homologues et/ou spécifiques et que l'on produit lesdites séquences pour la réalisation des sondes.

De préférence, l'amplification est effectuée à l'aide des deux amorces ou amplimères ayant respective-

EP 0 403 384 A2

ment les séquences suivantes :

5′ ACG TGG ACG AGG GCA TGC CC 3′
5′ TGT GCC ATG TAC AGC AGA GA 3′

Cette zone d'environ 110 à 115 bases peut également être définie par le fait que la zone d'hybridation de la seconde amorce est située juste avant le codon AUG d'initiation de la première phase de lecture du génome du Pestivirus.

De préférence, on recherche une séquence spécifique en dehors de la partie s'étendant entre sensiblement les bases 72 et 93 de la zone amplifiée.

De préférence, on recherche une séquence commune dans la partie s'étendant entre sensiblement les bases 72 et 93 de la zone amplifiée.

De préférence, on recherche des séquences communes et/ou spécifiques dans les séquences de plusieurs ou de la totalité des virus référencés A à S sur la figure 1.

L'invention a aussi pour objet les oligonucléotides obtenus par le procédé décrit ci-dessus.

L'invention a aussi pour objet les oligonucléotides dont la séquence est complémentaire des séquences communes et les oligonucléotides dont la séquence est complémentaire des séquences spécifiques, des différents génomes des 19 variétés de Pestivirus citées ci-dessus.

Les oligonucléotides concernés par l'invention comprennent les séquences suivantes :

Oligonucléotide 1 complémentaire des bases 93 à 72 de tous les Pestivirus de la figure 1 excepté les variétés I, R et S :

5′ GTG GGC CTC TGC AGC ACC CTA TCA GG 3′.

Oligonucléotide 2 complémentaire des bases 93 à 72 de la variété I :

5′ GTG GGC CTC TGC AGC GCC CTA TCA GG 3′.

Oligonucléotide 3 complémentaire des bases 93 à 68 des variétés R et S :

5′ GCA GGT CTC TGC TAC ACC CTA TCA GGC TGT 3′.

Oligonucléotide 4 complémentaire des bases 68 à 44 des variétés de virus de la peste porcine :

5′ ACT CCC ATC ACG TGG TGT GA 3′.

Oligonucléotide 5 complémentaire des bases 68 à 44 de la variété Border E :

5′ AAC CCC AAC ACA GTG AGG TGT 3′

Oligonucléotide 6 complémentaire des bases 68 à 44 des variétés Border F et G :

5′ AAC CCC AAC ACC ATT AGG TGT 3′

Oligonucléotide 7 complémentaire des bases 69 à 51 des variétés de BVD H et K :

5′ TAT TCG TAA CAG TCG GTT A 3′.

Oligonucléotide 8 complémentaire des bases 69 à 51 des variétés de BVD L, M, N et P :

5′ TAT TCG TAG CGG TTG GTT A 3′.

Oligonucléotide 9 complémentaire des bases 69 à 51 de la variété de BVD O :

5′ TAT TCG TAG CGG TTG GTC A 3′.

Oligonucléotide 10 complémentaire des bases 69 à 51 de la variété de BVD I :

5′ TAT TCG TAG CGG TCG GTT A 3′.

Oligonucléotide 11 complémentaire des bases 69 à 51 de la variété de BVD J :

5′ TAT TCG TAG CAG TTG ATC A 3′.

Oligonucléotide 12 complémentaire des bases 69 à 51 de la variété de BVD Q :

5′ TAT TCG TAG CGG TCT GTC T 3′.

On peut, bien entendu, remplacer certains nucléotides par des éléments utilisant des bases équivalentes, par exemple inosine, et ces constructions entrent, par simple équivalence dans le cadre de l'invention.

De préférence, les oligonucléotides comprennent au moins 10 et notamment au moins 15 à 17 bases consécutives de la séquence correspondante.

L'invention a encore pour objet les sondes préparées par le procédé de recherche et de préparation de sondes selon l'invention.

L'invention a également pour objet des sondes nucléiques générales et spécifiques comportant respectivement tout ou partie desdites séquences communes et spécifiques décrites ci-dessus.

Pour des raisons de sélectivité de l'hybridation, lesdites sondes nucléiques, de préférence, comportent au minimum 15 à 17 bases consécutives de la séquence oligonucléotidique correspondante. Avantageusement, elles comportent la totalité de ladite séquence.

Cependant, dans certains cas, des sondes utilisables peuvent comporter un nombre de base inférieur, par exemple de 10 à 12.

Pour leur détection, les sondes nucléiques selon l'invention peuvent être marquées selon toute technique connue. Elles peuvent par exemple être couplées à des radioisotopes.

Pour une détection par voie non-radioactive, les sondes nucléiques peuvent comporter des groupements additionnels, tels que des groupements amine, SH ou toute autre molécule, permettant de coupler un marqueur non radioactif.

On peut aussi envisager, de manière connue en soi, de greffer aux nucléotides des séquences connues identiques sur lesquelles viendra s'apparier un fragment nucléique de détection commun à toutes les sondes, par exemple un fragment nucléique couplé à un radioisotope.

De préférence, les sondes selon l'invention seront produites par synthèse chimique, par exemple selon la méthode des "Phosphoramidites" (Beaucage, S.L., et Caruthers, M.H. (1981), Tetrahedron Lett. 22, 1859), ou par toute autre méthode appropriée.

Pour la détection des Pestivirus par sonde, on pourra avantageusement utiliser un mélange contenant la sonde correspondante à l'oligonucléotide 1 et une sonde correspondant à l'un des oligonucléotides 2 et 3 ou, de préférence, un mélange de ces trois sondes.

La détection de virus de la Peste Porcine pourra être réalisée avec la sonde correspondant à l'oligonucléotide 4, celle de virus de la maladie de Border, avec un mélange des sondes correspondant aux oligonucléotides 5 et 6, et celle de BVD, avec un mélange des sondes correspondant aux oligonucléotides 7 à 12. Toutes les combinaisons sont bien entendu possibles.

Selon l'invention, l'hybridation moléculaire à l'aide des sondes selon l'invention pourra soit être effectuée sur un échantillon de liquide, par exemple sang ou sérum de l'animal, par exemple à l'aide de la méthode de Mickey S. Urdea, (1987) GENE 61, 253-264, soit sur un échantillon contenant l'ARN total de cellules de l'animal.

Selon l'invention, on pourra également recourir, au préalable, à une amplification de l'ARN afin d'augmenter la sensibilité de la détection (SAIKI et al., Science, 230, 1530-1534 (1985)).

L'invention a encore pour objet les amorces comportant respectivement les séquences suivantes:

5′ ACG TGG ACG AGG GCA TGC CC 3′

5′ TGT GCC ATG TAC AGC AGA GA 3′,

notamment destinées à la mise en oeuvre du procédé de recherche et de préparation de sondes et de l'étape d'amplification du procédé de détection des Pestivirus.

D'autres avantages et caractéristiques de l'invention apparaîtront à la lecture de la description suivante, faite à titre d'exemple non limitatif et en se référant à la figure 1 représentant la transcription ADN des séquences de génome de Pestivirus selon l'invention.

## I - DETECTION DANS LE SERUM DE L'ANIMAL :

On peut suivre le procédé décrit par Mickey S. Urdea et al. dans Gene, 61 (1978) 253-264.

On peut également appliquer le Protocole suivant :

Les acides nucléiques sont extraits du sérum comme décrit par D. Larzul et al (D. Larzul et al, Journal of Hepatology, 1987 ; 5/199-204).

- Le sérum est incubé pendant une heure à 70° C dans une solution :

25 mM acétate de sodium pH = 6,5

2,5 mM EDTA

0,5 % SDS (peut être remplacé par Nonidet P40 (Sigma))

12,5 μg/ml d'ADN de sperme de saumon (voir préparation plus loin)

2,5 mg/ml de Proteinase K (Boehringer, Mannheim)

- Ajouter un volume de phénol (voir préparation au point II1), agiter au vortex, centrifuger 3 minutes à 8000 g, puis récupérer la phase supérieure.

- Ajouter un volume d'éther diéthyle, agiter au vortex, éliminer la phase supérieure, incuber 30 minutes à 68° C pour éliminer les traces d'éther diéthyle.

- Ajouter de l'acétate de sodium (concentration finale 0,25 M), et 2,5 volumes d'éthanol. Laisser reposer au moins minutes à -70° C.

- Centrifuger 15 minutes à 8000 g, éliminer le surnageant, sécher le culot et resuspendre dans un volume minimal d'eau.

- Effectuer l'amplification selon le point III suivant.

- Effectuer la détection à l'aide du procédé de détection décrit par D. Larzul et al.

## II DETECTION SUR L'ARN TOTAL DE CELLULES DE L'ANIMAL

1 - Préparation de l'ARN cellulaire :

La préparation de l'ARN cellulaire est dérivée de la méthode décrite par Chomczinski, P. et Sacchi, N., Anal. Biochem. 162 : 156-159 (1987). a) Solutions :

- Solution dénaturante : thiocyanate de guanidine 4M, citrate de sodium 25 mM, sarcosyl 0,5 %, 2-mercaptoéthanol 0,1M.

Cette solution peut se conserver pendant 1 mois à la température de la pièce et la même solution sans 2-mercaptoéthanol se conserve dans les mêmes conditions pendant 3 mois.

- Acétate de sodium 2M, pH = 4.
- Phénol : Phénol distillé (conservé en aliquote à -20°C) équilibré peu avant l'emploi avec une solution de Tris-Hcl 50 mM (pH = 7,5), EDTA 5 mM (pH = 7,5) (utiliser une ampoule à décanter ; plusieurs équilibrages sont nécessaires). La solution finale e conserve 2 à 3 semaines à 4°C.
- Chloroforme/alcool iso-amylique (49: 1 en volume).
- Isopropanol.
- Ethanol absolu.
- Sarcosyl 10 %.
- PBS : KCl 27 mM, $KH_2PO_4$ 15 mM, $Na_2 HPO_4$ 32 mM, NaCl 137 mM.

b) Protocole :

1. Les volumes donnés dans ce protocole sont calculés pour la préparation d'ARN à partir d'un tapis cellulaire de 80 cm². Ces volumes seront ajustés proportionnellement pour d'autres surfaces de tapis cellulaires (jusqu'à l'étape 10 exclue).

2. Aspirer le milieu de culture et rincer le tapis de cellule avec 5 ml de PBS. Aspirer le surnageant.

3. Les cellules sont lysées à l'aide de 5 ml de solution dénaturante. Le mélange est transféré dans un tube de 15 ml stérile (bien nettoyer le fond de la boîte de culture avec la solution de lyse, la solution est souvent très visqueuse à ce stade).

4. On ajoute a cette solution, successivement et en agitant doucement par renversement du tube après l'addition de chaque réactif, 0,5 ml d'acétate de sodium 2M, 5 ml de phénol et 1 ml de chloroforme/alcool iso-amylique.

5. Refroidir sur glace pendant 15 minutes.

6. Centrifuger 20 minutes à 10000 g et à 4°C.

7. Récupérer la phase aqueuse (phase supérieure) en prenant soin de ne pas entraîner de phase inférieure et d'interphase, dans lesquelles se trouvent l'ADN et les protéines.

8. Ajouter 1 volume d'isopropanol, et laisser au moins 1 heure à -20°C.

9. Centrifuger 20 minutes à 10000 g et à 4°C.

10. Eliminer le surnageant et resuspendre le culot d'ARN dans 0,7 ml de solution dénaturante. Transférer le tout dans un tube stérile de 1,5 ml.

11. Faire précipiter l'ARN à l'aide d'un volume d'isopropanol. Laisser reposer au moins une heure à -20°C.

12. Centrifuger dans une centrifugeuse de table à 8000 g pendant 10 minutes et à 4°C.

13. Eliminer le surnageant, sécher le culot sous vide (Speed vac), et le resuspendre dans 200 µl d'eau stérile. On peut y ajouter du sarcosyl (0,5 %) pour aider à la mise en suspension du culot. Si le culot reste difficile à resuspendre, chauffer 10 minutes à 65°C et agiter au Vortex plusieurs fois à raison de 15 secondes chaque. Répéter ce traitement une fois si nécessaire.

14. Ajouter 200 µl de phénol, agiter par renversement du tube, puis ajouter 200 µl de chloroforme/alcool isoamylique et agiter de même.

15. Centrifuger 3 minutes dans une centrifugeuse de table (8000g, 4°C) et récupérer la phase aqueuse (phase supérieure). Ajouter 1 volume de chloroforme/alcool isoamylique, agiter par renversement du tube et centrifuger comme précédemment.

16. Récupérer la nouvelle phase aqueuse, ajouter de l'acétate de sodium (concentration finale 0,25 M) et 2,5 volumes d'éthanol. Laisser reposer au moins 20 minutes à -70°C.

17. Centrifuger 15 minutes dans une centrifugeuse de table, à 8000 g, éliminer le surnageant. Sécher le culot sous vide (Speed vac) et resuspendre dans un volume minimum d'eau stérile.

18. Evaluer la quantité d'ARN obtenue par lecture de la densité optique à 260 nm :

Concentration d'ARN (en µg/ml) = DO 260 (1 cm)/0,024.

On obtient généralement 200 µg d'ARN par tapis de cellules confluentes de 80 cm².

19. Ajuster la concentration en ARN à 3,3 µg/µl avec de l'eau stérile.

2 - Détection de l'ARN viral :

A - Fixation de l'ARN sur la nitrocellulose (ou tout autre support approprié : nylon, etc) :

a) Solutions :
- SSC 20x : Dissoudre 175,3 g de NaCl et 88,2 g de citrate de sodium dans 800 ml d'H$_2$O. Ajuster le pH à 7,0 avec quelques gouttes de NaOH 10N. Ajuster le volume à 1 litre.
- Aldéhyde formique (37 %) en solution.

b) Protocole :
1. A 3 µl (10 µg) d'ARN total de cellules, ajouter 0,3 µl d'aldéhyde formique à 37 % et chauffer 20 minutes à 60°C. Maintenir à 0°C sur de la glace.

2. Déposer une feuille de nitrocellulose 5 minutes dans du SSC 20x puis laisser sécher la nitrocellulose 10 minutes à la température ambiante sur un papier buvard.

3. Déposer les 3,3 µl de la solution d'ARN sur la nitrocellulose ainsi traitée (la tache ne doit pas dépasser 1 cm$^2$ environ).

4. Rincer la nitrocellulose 2 minutes dans une solution de SSC 6x et laisser sécher à la température ambiante pendant 15 minutes.

5. Cuire la nitrocellulose deux heures à 80°C et sous vide. L'ARN ainsi déposé sur la nitrocellulose est stable plusieurs semaines dans un dessicateur à température ambiante (entre deux filtres de papier whatman 3MM).

B - Hybridation.

a) Solutions.
- Solution de préhybridation :
SSC 4x (voir plus haut),
Denhart 6x (0,12 % poids/volume (p/v) de Ficoll 400, 0,12 % p/v de PVP (polyvinylpyrrolidone), 0,12 % p/v de fraction V de sérum-albumine bovine),
0,1 % p/v de sodium dodécyl sulfate,
300 µg/ml d'ADN de sperme de saumon dénaturé (voir point b ci-après).
- Solution d'hybridation :
SSC 4x
Denhart 2x (0,04 % p/v de Ficoll 400, 0,04 % p/v de PVP, 0,04 % p/v de fraction V de sérum-albumine bovine),
3 % p/v de sodium dodécyl sulfate,
10 % p/v de sulfate de dextrane (par dilution d'une solution à 50 % préparée à l'avance),
200 µg/ml d'ADN de sperme de saumon dénaturé (voir point b ci-après),
50 ng/ml de sonde marquée, chauffée 10 minutes à 100°C.
- Solution I : SSC 2x, 0,1 % p/v de sodium dodécyl sulfate.
- Solution J : SSC 0,25x, 0,1 % p/v de sodium dodécyl sulfate.
- TE : 10 mM Tris-HCl, pH=7,5, EDTA 1 mM, pH=7,5.

b) Préparation de l'ADN de sperme de saumon.
1. Dissoudre la nuit, à température ambiante, 1 g d'ADN de sperme de saumon dans 100 ml de NaOH 0,4 M.

2. Porter à ébullition pendant 45 minutes.

3. Refroidir sur glace et amener à pH=7 avec de l'acide acétique glacial.

4. Centrifuger à 4000 g pendant 10 minutes et à température ambiante. Récupérer le surnageant.

5. Ajouter 2 volumes d'éthanol absolu, laisser reposer au moins 60 minutes à -20°C.

6. Centrifuger à 8000g pendant 15 minutes et à température ambiante. Jeter le surnageant.

7. Sécher le culot sous vide

8. Le dissoudre dans 50 ml de TE.

9. Evaluer la quantité d'ADN par lecture de la densité optique à 260 nm :
Concentration en ADN (en µg/ml) = DO 260 (1 cm)/0,02.
Ajuster la concentration à 10 mg/ml.

10. L'ADN de sperme de saumon ainsi préparé se conserve plusieurs mois à -20°C.

c) Protocole.
1. Chauffer la nitrocellulose au moins 4 heures à 55°C (cf point B-d/ ci-après), dans 1 ml/cm$^2$ de

la solution de préhybridation.

Il vaut mieux individualiser les filtres dans des sacs en matière plastique. Bien chasser les bulles d'air avant de refermer à chaud lesdits sacs.

2. Vider le sac et ajouter 0,5 ml/cm² de la solution d'hybridation. Chauffer au moins 12 heures à 55° C (cf point B-d/).

3. Vider le sac, l'ouvrir et laver le filtre à température ambiante 4 fois 5 minutes dans la solution I.

4. Laver le filtre à température ambiante 4 fois 5 minutes dans la solution J.

5. Laver le filtre, à 45° C (cf point B-d/), 3 fois 15 minutes dans la solution J.

6. Révéler suivant le type de marquage de la sonde.

d) Températures.

Les températures citées au protocole (point B-c/) dépendent de la TM de la sonde (TM = température pour laquelle 50 % de la sonde est passée de la forme hybridée à la forme non hybridée).

L'effet de la composition de base sur la TM est plus important lorsque les sondes ont moins de 50 bases (MEINKOTH, J. ET WAHL, G., ANAL. BIOCHEM., 138, p 267-284, 1984). Pour des sondes oligonucléotidiques comportant jusqu'à 20 bases, on peut, lorsque l'hybridation est effectuée dans des conditions standard (NaCl 0,9 M environ), déterminer une valeur de TM approximative en utilisant la formule suivante (WALLACE, R.B. et al., Nucl. Acids Res., 6, P. 3543-3656, 1979) :

$$TM\ (°C) = 4\ (G + C) + 2\ (A + T)$$

dans laquelle G, C, A et T correspondent au nombre de bases correspondantes (Guanidine, Cytosine, Adénine, Thymine) dans l'oligonucléotide.

En hybridation sur filtre (c'est-à-dire une hybridation avec un échantillon d'acide nucléique immobilisé sur une membrane) à l'aide de sondes oligonucléotidiques, on devrait pouvoir employer une température de 5° C inférieure pour hybrider parfaitement les séquences. Lorsque l'on utilise ces courtes sondes, pour chaque paire de bases non hybridée, une réduction supplémentaire de température de 5° C est nécessaire pour conserver la stabilité de l'hybridation.

3 - Détection de l'ARN amplifié.

L'ARN total de cellules de l'animal à subi une amplification selon le procédé décrit au point III qui suit.

Au cours de cette amplification, l'ARN a été transcrit en ADN, de sorte qu'il s'agit ici d'une hybridation ADN-ADN dont le protocole ne diffère que pour le point II A-b/, qui devient :

Chauffer 10 µl de l'amplification à 95° C pendant 5 minutes.

Déposer sur glace.

Déposer une feuille de nitrocellulose 5 minutes dans SSC 20x puis laisser sécher la nitrocellulose 10 minutes à température ambiante sur un papier buvard.

Déposer les 10 µl sur la nitrocellulose ainsi traitée, à l'aide d'un appareil commercial de "Dot Blotting".

Cuire la nitrocellulose 2 heures à 80° C sous vide.

III - AMPLIFICATION DE L'ARN.

L'amplification permet d'amplifier des séquences d'acides nucléiques en hybridant des amorces et en synthétisant le brin complémentaire de la séquence depuis l'amorce en présence de nucléotides triphosphates et d'ADN polymérase ou autres enzymes de polymérisation, de telle sorte que le produit d'extension de l'amorce serve de matrice pour la synthèse de la séquence et ainsi de suite.

L'amplification met en oeuvre deux amorces, chacune d'elles allant s'hybrider sur son site spécifique sur son brin d'ADN complémentaire

- amorce A : 5' ACG TGG ACG AGG GCA TGC CC 3'

(complémentaire de la base 234 à la base 254 du génome de BVD Osloss),

- amorce B : 5' TGT GCC ATG TAC AGC AGA GA 3'

(complémentaire de la base 385 à la base 365).

1 - Hybridation de l'amorce pour la transcriptase inverse (amorce B).

- on prélève de 1 à 8 µl de la solution d'ARN (10 à 20 µg),

- on ajoute 20 ng de l'oligonucléotide servant d'amorce,

- on amène à une concentration finale de

250 mM KCl

2,5 mM Tris-HCl pH = 7

0,25 mM EDTA

dans un volume final de 10 $\mu$l,

    - on chauffe pendant 15 minutes à 65°C, puis

    - 60 minutes à température ambiante (ou une température plus élevée).

    Cette température est déterminée par la séquence de l'oligonucléotide et par la concentration en sel du milieu, dans le cas présent 0,25 M. Différentes méthodes existent pour déterminer cette température :

- MEINKOTH, J. and WAHL, G. Anal. Biochem. 138, 267-284, 1984,

- WALLACE, R.B. et al., Nucl.Acids Res., 6, 3543-3656, 1979,

- GOEDDEL, D.V. et al., Nature, 287, 411-416, 1980,

- SUGGS, S.V. et al., Proc. Natl. Acad. Sci. USA, 78, 6613-6617, 1981,

- SZOSTAK, J.W. et al., Methods Enzymol., 68, 419-428, 1979,

- SUGGS, S.V. et al., ICN-UCLA Symp. Dev. Biol., 23, 683-693, 1981

## 2 - Synthèse du brin d'ADN par la transcriptase inverse.

    - on ajoute 20 unités de transcriptase inverse M-MLV : clone M-MLV (Moloney Murine Leukemia Virus) de la transcriptase inverse, de Bethesda Research Laboratories ;

    - on ajuste à une concentration finale de :

7 mM Tris-HCl PH = 8,6

7 mM MgCl$_2$

3,5 mM DTT (Dithiothréitol)

60 $\mu$M dATP, dTTP, dGTP, dCTP

5 ng/$\mu$l d'amorce B

75 mM KCl

dans un volume final de 33 $\mu$l ;

    - on laisse incuber 15 minutes à 22°C, puis 45 minutes à 35°C.

## 3 - Amplification.

    - on amène la solution à une concentration de :

50 mM KCl

10 mM Tris-HCl pH = 8,6

2,5 mM MgCl$_2$

200 $\mu$g/ml Gélatine

200 $\mu$M dNTP

10 ng/$\mu$l d'amorce A

10 ng/$\mu$l d'amorce B

dans un volume final de 100 $\mu$l ;

    - on ajoute 4 unités de TAQ (Thermus Aquaticus DNA Polymerase de New England Biolabs, ref. : KALEDIN, A.S. et al., Biokhimia 45 (4), 644-651, 1980) ;

    - on chauffe 360 secondes à 93°C ;

       A) on chauffe 60 secondes à 93°C ;

       B) on amène à 22°C pendant 120 secondes ou à une température plus élevée, et/ou pendant un temps plus important selon le cas ;

       C) on incube 90 secondes à 72°C ;

    - on répète de A) à C) 4 fois ;

    - on répète ensuite de 25 à 30 fois le cycle de température ci-après

       D) 60 secondes à 93°C ;

       E) 90 secondes à 55°C ;

       F) 90 secondes à 72°C.

    L'étape d'amplification peut être avantageusement menée dans un appareil tel que celui décrit dans la demande de brevet européen EP-A-0.236.069.

| Annexe | | | |
|---|---|---|---|
| Ref. | Souche | Origine | Biotype |
| Peste porcine | | | |
| A | A19 | Rhône Mérieux 1965 | non cytopathogène |
| B | Thiverval | Launais et al. (1972)[4] | non cytopathogène |
| C | C30-chinoise | Rhône Mérieux 1970 | non cytopathogène |
| D | Alfort | Aynaud (1968)[1] | non cytopathogène |
| Maladie de Border | | | |
| E | AV-Aveyron | Rhône Mérieux 1984 | non cytopathogène |
| G - F | BD (c/nc) | Moredun 1983 | cytopathogène |
| BVD | . | | |
| H | Singer (c/nc) | Mc. Clurkin & Coria (1978)[5] | cytopathogène |
| I | Lamspringe | Liess (1967) | cytopathogène |
| J | Oregon | Gillepsie et al. (1960)[2] | cytopathogène |
| K | NADL | Gutekunst (1963)[3] | cytopathogène |
| L | New-York | ATCC | non cytopathogène |
| M-N | Osloss (c/nc) | Liess | cytopathogène |
| O à S | Souches de terrain | | |

1. Aynaud, J.M., 1968. Etude de la multiplication en cycle unique d'un clone du virus de la peste porcine classique au moyen de l'immunofluorescence. Ann. Rech. Veter. 1:25-36.

2. Gillespie, J.H., Baker, J.A. and McEntee, K., 1960. A cytopathic strain of virus diarrhea virus. Cornell Vet. 50:73-79.

3. Gutekunst, D.E. and Malmquist,W.A., 1963. Separation of a soluble antigen and infectious particles of Bovine Virus Diarrhea virus and their relationship to Hog Cholera. Can. J. Comp. Med. and Vet. Sc. 27:n°5.

4. Launais, M., Aynaud, J.M. and Corthier, G., 1972. Swine fever virus : properties of a clone (Thiverval strain) isolated in cell culture at low temperature. Use in vaccination. Rev. Med. Veter. 123:1537-1554.

5. McClurkin, A.W. and Coria, M.F., 1978. Selected isolates of bovine viral diarrhea (BVD) virus propagated on bovine turbinate cells : virus titre and soluble antigen production as factors in immunogenicity of killed BVD virus. Arch. Virol. 58:119-128.

## Revendications

1. Procédé de recherche et de préparation de sondes communes et/ou spécifiques des variétés de Pestivirus, caractérisé en ce que l'on amplifie une zone d'environ 110 à 115 bases située vers l'extrémité 5' sur le génome d'un Pestivirus dans la région non codante correspondant à la région située entre les bases 200 et 310 du génome du virus BVD Osloss à l'aide d'amorces convergentes s'hybridant sensiblement au niveau des extrémités de cette zone non codante, que l'on séquence la zone amplifiée et que, par comparaison aux zones similaires déjà séquencées d'autres variétés de Pestivirus, on recherche les séquences oligonucléotidiques homologues et/ou spécifiques, et que l'on produit lesdites séquences pour la réalisation des sondes.

2. Procédé selon la revendication 1, caractérisé en ce que l'on utilise les deux amorces ayant respectivement les séquences suivantes :
5' ACG TGG ACG AGG GCA TGC CC 3'
5' TGT GCC ATG TAC AGC AGA GA 3'.

3. Procédé selon l'une des revendications 1 et 2, caractérisé en ce que l'on recherche une séquence spécifique en dehors de la partie s'étendant entre sensiblement les bases 72 et 93 de la zone amplifiée.

4. Procédé selon l'une des revendications 1 et 2, caractérisé en ce que l'on recherche une séquence commune dans la partie s'étendant entre sensiblement les bases 72 et 93 de la zone amplifiée.

5. Procédé selon l'une des revendications 3 et 4, caractérisé en ce que l'on recherche les séquences communes et/ou spécifiques dans les séquences de plusieurs ou de la totalité des virus référencés A à S sur la figure 1.

6. Oligonucléotides obtenus par le procédé selon l'une quelconque des revendications 1 à 5.

7. Oligonucléotide comprenant la séquence suivante :
5' GTG GGC CTC TGC AGC ACC CTA TCA GG 3'.

8. Oligonucléotide comprenant la séquence suivante :
5' GTG GGC CTC TGC AGC GCC CTA TCA GG 3'.

9. Oligonucléotide comprenant la séquence suivante :
5' GCA GGT CTC TGC TAC ACC CTA TCA GGC TGT 3'.

10. Oligonucléotide comprenant la séquence suivante :
5' ACT CCC ATC ACG TGG TGT GA 3'.

11. Oligonucléotide comprenant la séquence suivante :
5' AAC CCC AAC ACA GTG AGG TGT 3'.

12. Oligonucléotide comprenant la séquence suivante :
5' AAC CCC AAC ACC ATT AGG TGT 3'.

13. Oligonucléotide comprenant la séquence suivante :
5' TAT TCG TAA CAG TCG GTT A 3'.

14. Oligonucléotide comprenant la séquence suivante :
5' TAT TCG TAG CGG TTG GTT A 3'.

15. Oligonucléotide comprenant la séquence suivante :
5' TAT TCG TAG CGG TTG GTC A 3'.

16. Oligonucléotide comprenant la séquence suivante :
5' TAT TCG TAG CGG TCG GTT A 3'.

17. Oligonucléotide comprenant la séquence suivante :
5' TAT TCG TAG CAG TTG ATC A 3'.

18. Oligonucléotide comprenant la séquence suivante :
5' TAT TCG TAG CGG TCT GTC T 3'.

19. Oligonucléotide selon 1 une quelconque des revendications 7 à 18, caractérisé en ce qu'il comporte au moins 10 et notamment au moins 15 à 17 bases consécutives de la séquence correspondante.

20. Sondes préparées par le procédé selon l'une quelconque des revendications 1 à 5.

21. Sonde ADN pour la détection de Pestivirus par hybridation moléculaire, caractérisée en ce qu'elle comprend tout ou partie de la séquence de la revendication 7, 8 ou 9.

22. Sonde ADN pour la détection du virus de la peste porcine par hybridation moléculaire, caractérisée en ce qu'elle comprend tout ou partie de la séquence selon la revendication 10.

23. Sonde ADN pour la détection du virus de la maladie de Border par hybridation moléculaire, caractérisée en ce qu'elle comprend tout ou partie de la séquence selon la revendication 11 ou 12.

24. Sonde ADN pour la détection du virus BVD par hybridation moléculaire, caractérisée en ce qu'elle comprend tout ou partie de la séquence selon l'une des revendications 13 à 18.

25. Mélange comprenant au moins deux types de sondes ADN selon la revendications 21.

26. Mélange comprenant les deux sondes ADN selon la revndication 23.

27. Mélange comprenant au moins deux types de sondes ADN selon la revendication 24.

28. Sonde ADN selon l'une quelconque des revendications 21 à 27, caractérisée en ce qu'elle comprend au moins 10 et notamment au moins 15 à 16 bases consécutives de la séquence correspondante.

29. Sonde ADN selon l'une quelconque des revendications 21 à 28, caractérisée en ce qu'elle est obtenue par synthèse chimique.

30. Procédé de détection de virus du genre Pestivirus par hybridation moléculaire, caractérisé en ce que l'on applique la sonde ou le mélange selon l'une des revendications 1 à 5 ou 21 à 29 sur un échantillon de liquide de l'animal testé.

31. Procédé de détection de virus du genre Pestivirus par hybridation moléculaire, caractérisé en ce que l'on applique la sonde ou le mélange selon l'une des revendications 1 à 5 ou 21 à 29 sur un échantillon contenant l'ARN total de cellules de l'animal testé.

32. Procédé selon la revendication 30 ou 31, caractérisé en ce que, avant l'hybridation, on procède à une amplification de l'ARN viral à l'aide d'une paire d'amorces appropriées.

33. Amorces notamment pour la mise en oeuvre des procédés selon l'une quelconque des revendications 1 à 5 ou 32, caractérisées en ce qu'elles comportent respectivement les séquences suivantes:
5' ACG TGG ACG AGG GCA TGC CC 3'
5' TGT GCC ATG TAC AGC AGA GA 3'.

Figure 1 (feuilles 1 et 2)

| | | | 1 | 44 | 51 | 60 |
|---|---|---|---|---|---|---|
| PESTE PORCINE | A | Ppca19.Frg | aagaCAcaccTTAa.CCt.aGCgGGGGTcGctagGg.tgAAAtca.....caccacgtGa | | | |
| | B | Ppccog1a.Frg | aagaCAcaccTTAa.CCt.aGCgGGGGTcGctagGg.tgAAAtca.....caccacgtGa | | | |
| | C | Ppcc30.Frg | aagaCAcaccTTAa.CCt.aGCgGGGGTcGctagGgatgAAAtca.....caccacgtGa | | | |
| | D | Ppcppl.Frg | aagaCAcaccTTAa.CCt.aGCgGGGGTcGctagGgg.gAAAtca.....caccacgtGa | | | |
| BORDER | E | Bdav.Frg | atgaCAcgctTTAggCC..gGCaGGGGTcGcc.gGgtcgAAAaca..cctcactg..tGt | | | |
| | F | Bdbdnc.Frg | aagaCAtgctTTAatCCt.gGCgGGGGTcGccagGg.tgAAAaca..cctaatgg..tGt | | | |
| | G | Bdbdc.Frg | aagaCAtgctTTAatCCt.gGCgGGGGTcGccagGg.tgAAAaca..cctaatgg..tGt | | | |
| BVD | H | Bvdsgnc.Frg | aaagCAcatcTTAa.CCtgaGCgGGGGTcGcccaGg.taAAAgcagttctaaccgactGt | | | |
| | I | Bvdlamc.Frg | acagCAcatcTTAa.CCtgaGCgGGGGTcGctcaGg.cgAAAacggt.ctaaccgaccGc | | | |
| | J | Bvdmd1.Frg | aaagCAcatcTTAa.CC.gaGCgGGGGTcGctcgGa.caAAAacagt.ttgatcaactGc | | | |
| | K | Bvdnad1.Frg | aaagCAcatcTTAa.CCtgaGCgGGGGTcGcccaGg.taAAAgcagttttaaccgactGt | | | |
| | L | Bvdny.Frg | acagCAcatcTTAa.CCtgaGCgGGGGTcGttcaGg.tgAAAacggt.ttaaccaaccGc | | | |
| | M | Bvdosnc.Frg | acagCAcatcTTAa.CCtgaGCgGGGGTcGttcaGg.tgAAAgcggt.ttaaccaaccGc | | | |
| | N | Bvdosc.Frg | acagCAcatcTTAa.CCtgaGCgGGGGTcGttcaGg.tgAAAgcggt.ttaaccaaccGc | | | |
| | O | Vp123.Frg | acagCAcatcTTAa.CCtgaGCgGGGGTcGctcaGg.cgAAAacggt.ttgaccaaccGc | | | |
| | P | Ve48nc.Frg | acagCAcatcTTAa.CCtggGCgGGGGTcGttcaGg.tgAAAacggt.ttaaccaaccGc | | | |
| | Q | 88753.Frg | acagCAcatcTTAg.CCtgaGCgGGGGTcGcccaGg.tgAAAgcggtgaagacagaccGc | | | |
| | R | Sgcinc1.Frg | acggCAcatcTTAa.CCtatGCgGGGGTtGcatgGg.tgAAA..ggcccattcgtggcGt | | | |
| | S | Sgcinc2.Frg | tcggCAcatcTTAa.CCtatGCgGGGGTtGcatgGg.tgAAAgc.gc.cattcgtggcGt | | | |
| | | Consensus * | ----CA----TTA--CC---GC-GGGGT-G----G----AAA---------------G- | | | |

* Consensus = séquence commune à tous les génomes.

EP 0 403 384 A2

Figure 1 (feuille 2)

```
              61     68   72                                    93                    119
Ppca19.Frg    TggGagtACgaCCTGATAGGGtGctGCAGAGgCCcaC..tattaggctagTA.tAAaaa
Ppccogla.Frg  TggGagtACgaCCTGATAGGGtGctGCAGAGgCCcaC..tattaggctagTA.tAAaaa
Ppcc30.Frg    TggGagtACgaCCTGATAGGGtGctGCAGAGgCCcaC..tattaggctagTA.tAAaàa
Ppcppl.Frg    TggGagtACgaCCTGATAGGGtGctGCAGAGgCCcaC..tattaggctagTA.aAAaaa
Bdav.Frg      TggGgttACagCCTGATAGGGtGctGCAGAGgCCcaC.gcat.aagctagTA.tAAaaa
Bdbdnc.Frg    TggGgttACagCCTGATAGGGtGctGCAGAGgCCcaC.gtat.aggctagTA.tAAaaa
Bdbdc.Frg     TggGgttACagCCTGATAGGGtGctGCAGAGgCCcaC.gtat.aggctagTAtaAAaaa
Bvdagnc.Frg   TacGaatACagCCTGATAGGGtGctGCAGAGgCCcaCtgtt.....ctgcTActAAaaa
Bvdlamc.Frg   TacGaatACagCCTGATAGGGcGctGCAGAGgCCcaCtgta.....ttgcTActAAaaa
Bvdmdl.Frg    TacGaatACagCCTGATAGGGtGctGCAGAGgCCcaCtgta.....ttgcTActAAaaa
Bvdnadl.Frg   TacGaatACagCCTGATAGGGtGctGCAGAGgCCcaCtgta.....ttgcTActAAaaa
Bvdny.Frg     TacGaatACagCCTGATAGGGtGctGCAGAGgCCcaCtgta.....ttgcTActAAaaa
Bvdosnc.Frg   TacGaatACagCCTGATAGGGtGctGCAGAGgCCcaCtgta.....ctgcTActAAaaa
Bvdosc.Frg    TacGaatACagCCTGATAGGGtGctGCAGAGgCCcaCtgta.....ttgcTActAAaaa
Vp123.Frg     TacGaatACagCCTGATAGGGtGctGCAGAGgCCcaCtgta.....ttgcTActAAaaa
Ve48nc.Frg    TacGaatACagCCTGATAGGGtGctGCAGAGgCCcaCtgta.....ttgcTActAAaaa
88753.Frg     TacGaatACagCCTGATAGGGtGctGCAGAGgCCcaCtgta.....atgcTActAAaaa
Sgcincl.Frg   TatGgacACagCCTGATAGGGtGtaGCAGAGaCCtgC..tatt...ccgcTAgtAAaa.
Sgcinc2.Frg   TatGgacACagCCTGATAGGGtGtaGCAGAGaCCtgC..tatt...ccgcTAgtAA...
Consensus     T--G---AC--CCTGATAGGG-G--GCAGAG-CC--C------------TA--AA---
```

EP 0 403 384 A2